Europäisches Patentamt

European Patent Office

Office européen des brevets

(19)

(11) Veröffentlichungsnummer: **0 228 523 B1**

(12) **EUROPÄISCHE PATENTSCHRIFT**

(45) Veröffentlichungstag der Patentschrift: 07.08.91

(51) Int. Cl.⁵: **A61F 2/36**, A61F 2/46

(21) Anmeldenummer: **86114938.3**

(22) Anmeldetag: **28.10.86**

(54) **Hüftgelenkprothese.**

(30) Priorität: **29.10.85 DE 3538346**

(43) Veröffentlichungstag der Anmeldung:
**15.07.87 Patentblatt 87/29**

(45) Bekanntmachung des Hinweises auf die
Patenterteilung:
**07.08.91 Patentblatt 91/32**

(84) Benannte Vertragsstaaten:
**FR GB IT**

(56) Entgegenhaltungen:
**DE-A- 3 412 362**
**GB-A- 945 292**
**US-A- 2 682 265**
**US-A- 3 704 707**

(73) Patentinhaber: **Kliefoth, Ingo**
**Schulberg 2**
**W-2411 Kühsen(DE)**

(72) Erfinder: **Kliefoth, Ingo**
**Schulberg 2**
**W-2411 Kühsen(DE)**

(74) Vertreter: **Heldt, Gert, Dr. Dipl.-Ing.**
**Neuer Wall 59 III**
**W-2000 Hamburg 36(DE)**

## Beschreibung

Die Erfindung betrifft eine Hüftgelenkprothese mit einer Kappe, die eine in einer Hüftpfanne verschwenkbare lagerbare Gleitfläche aufweist und mit der Kräfte auf einen der Hüftpfanne zugewandten Hüftkopf eines Oberschenkelknochens übertragbar sind.

Aus der US-A-2 682 265 ist eine Hüftgelenkprothese bekannt, die eine in einer Hüftpfanne verschwenkbar lagerbare Gleitfläche aufweist. Bei dieser Hüftgelenkprothese handelt es sich aber um eine den Hüftkopf ersetzende Prothese. Bei dieser wird ein gesamter Hüftkopf auf eine Fläche aufgesetzt, auf der der Hüftkopf mit einer planparallel zu seinem Querschnitt verlaufenden Auflagefläche aufliegt. Auf diese Auflagefläche wird der Hüftkopf mit einer Zähnelung befestigt, die an einer Auflegescheibe vorgesehen ist. Darüberhinaus wird der Hüftkopf an einem mehrfach unterteilten Bolzen befestigt, der sich durch ei ne den Femurknochen durchlaufende Bohrung erstreckt. Dieser mehrfach unterteilte Bolzen besitzt keilförmig miteinander verbindbare Teilstrecken, auf denen der Hüftkopf fest verkeilt wird. Eine Nachgiebigkeit des Bolzens in Längsrichtung der Bolzenachse ist nicht vorgesehen.

Eine derartig feste Verkeilung des Hüftkopfes ist für Kappenprothesen nicht brauchbar.

Aufgabe der vorliegenden Erfindung ist es daher, eine Hüftgelenkprothese mit einer Kappe, die eine in einer Hüftpfanne verschwenkbar lagerbare Gleitfläche aufweist, so zu verbessern, daß ein Verrutschen der Kappe verhindert wird.

Diese Aufgabe wird erfindungsgemäß dadurch gelöst, daß die Kappe eine der Gleitfläche abgewandt angeordnete und auf einem Hüftkopf eines Oberschenkelknochens abstützbare Basis und eine in Richtung der von der Kappe zu übertragenden Kräfte nachgiebige Verankerung aufweist, die als ein Schaft ausgebildet ist, der in einer sich in Richtung der zu übertragenden Kräfte verlaufenden Führung verschieblich gelagert ist.

Bei einer derartigen Hüftgelenkprothese kann die Kappe einem sich unter ihr zurückbildenden Femurknochen nachgeben, ohne daß deswegen die Führung der Kappe auf dem Femurknochen beeinträchtigt wird. Die Kappe kann bezüglich ihrer ursprünglich gewollten Lage nicht verrutschen. Sie behält vielmehr ihre bezüglich der Hüftpfanne optimale Lage bei und wird daher sicher in der Hüftpfanne geführt. Andererseits erzeugt sie den für die Erhaltung des Femurknochens notwendigen Druck.

Durch die Verankerung wird gewährleistet, daß die Kappe auch dann nicht vom Hüftkopf abgleitet, wenn sich das Knochengewebe des Hüftkopfes zurückgebildet hat. Vielmehr kann sich die Kappe weitgehend flexibel den jeweiligen Hüftkopfkonstellationen anpassen. Auf diese Weise kann sich die Kappe als Überträger der Traglast physiologisch richtig der jeweiligen Entwicklung der Druckbelastung anpassen. Sollte sich mithin unter der Kappe eine Knochenatrophie ausbilden, so kann die Kappe in Richtung der Druckbelastung dem gesunden Knochengewebe nachrutschen und sich auf diesem jeweils festsetzen. Darüber hinaus kann damit gerechnet werden, daß durch die ständig gleichbleibende Belastung des Hüftkopfes die Knochenatrophie sich stark verlangsamt, möglicherweise sogar zum Stillstand kommt. Auf jeden Fall wird verhindert, daß die Kappe vom Hüftkopf abrutscht, unabhängig davon, ob ein Knochenschwund eintritt oder nicht.

Damit ist für eine sichere Funktion der Endoprothese gesorgt, ohne daß die Kappe in eine Stellung rutschen kann, in der sie die Funktion des Hüftgelenkes behindern kann.

Weitere Einzelheiten der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

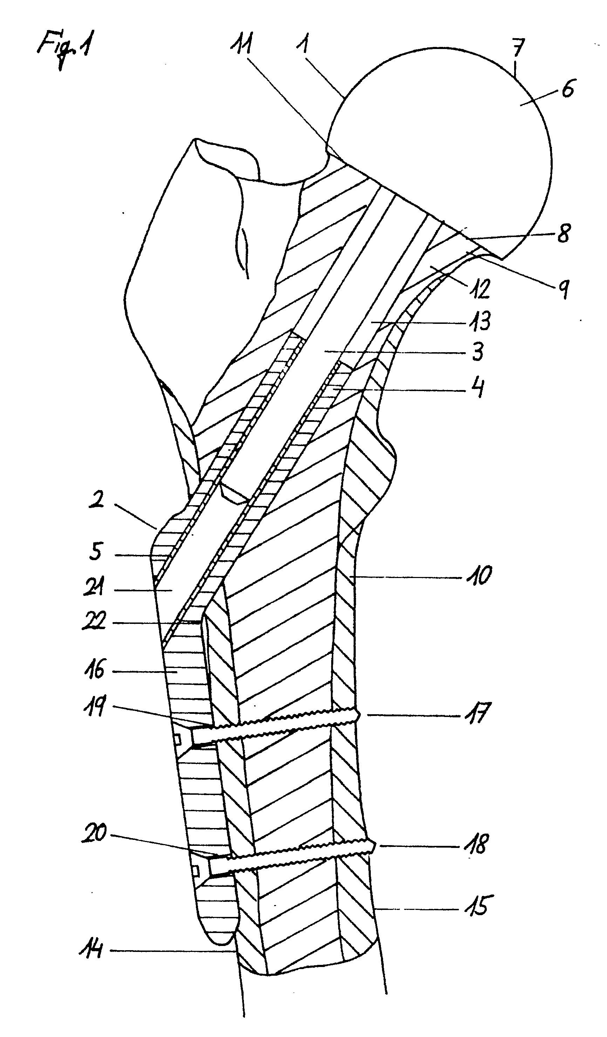

Fig. 1: einen Längsschnitt durch einen Oberschenkelknochen und

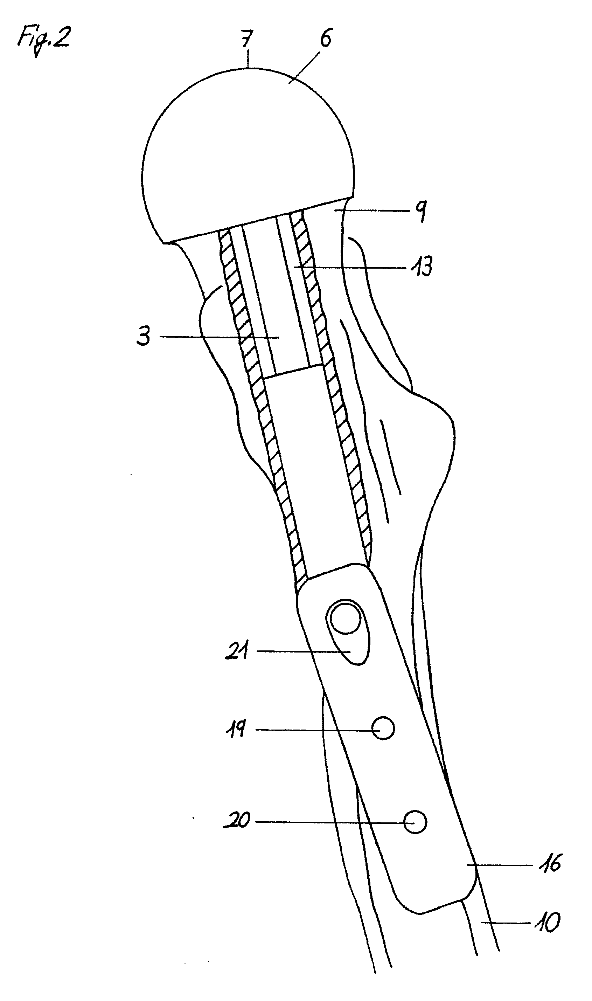

Fig. 2: eine Draufsicht auf eine dem Hüftkopf abgewandte Längsseite eines Oberschenkelknochens.

Eine Hüftgelenkprothese besteht im wesentlichen aus einem Kopfteil (1) und einem Führungsteil (2). Der Kopfteil ragt mit einem Bolzen (3) in eine am Führungsteil (2) befestigte Hülse (4) hinein. Der Bolzen (3) ist in der Hülse (4) verschieblich gelagert. Zu diesem Zwecke ist innerhalb der Hülse (4) eine Innenauskleidung (5) vorgesehen, die sich in Längsrichtung der Hülse (4) erstreckt und deren Innendurchmesser einem Außendurchmesser des Bolzens (3) entspricht. Der Bolzen (3) sowie die Hülse (4) können einen kreisförmigen oder eckigen Querschnitt aufweisen.

Der Bolzen (3) trägt auf seinem der Hülse (4) abgewandten Ende eine Kappe (6), deren dem Bolzen (3) abgewandte Oberfläche als eine Gleitfläche (7) ausgebildet ist, mit der die Kappe (6) in einer nicht dargestellten Hüftpfanne gelagert ist, die in einem ebenfalls nicht dargestellten Beckenknochen befestigt ist. Die Kappe (6) wird auf ihrer der Gleitfläche (7) abgewandten Unterseite von einer Basis (8) abgeschlossen, auf der sich der Bolzen erhebt. Mit dieser Basis (8) liegt die Kappe (6) auf einem Hüftkopf (9) auf, der an einem Oberschenkelknochen (10) an seinem dem Beckenknochen zugewandten Ende ausgebildet ist. Die Kappe (6) ist als Ausschnitt einer Hohlkugel ausgebildet, in deren Zentrum der Bolzen (3) befestigt ist. Die Basis (8) der Kappe (6) liegt auf einer Auflagefläche (11) auf, die zu diesem Zwecke an dem Hüftkopf (9) angefräst ist.

Durch den Hüftkopf (9) und einen sich an ihn in Richtung des Bolzens (3) anschließenden Schenkelhals (12) erstreckt sich eine Bohrung (13), in der der Bolzen (3) geführt ist. Diese Bohrung (13) erhebt sich in etwa rechtwinklig auf einer von der Auflagefläche (11) aufgespannten Ebene. Diese Bohrung (13) mündet an einer dem Hüftkopf (9) gegenüberliegenden Längsfläche (14) des Oberschenkelknochens (10). Diese Längsfläche (14) begrenzt einen Oberschenkelschaft, der sich unmittelbar im Anschluß an den Schenkelhals (12) in Richtung auf ein dem Hüftkopf (9) abgewandtes Ende (30) des Oberschenkelknochens (10) erstreckt.

Von der Längsfläche (14) erstreckt sich in Richtung der Bohrung (13) die Hülse (4). Sie ist außerhalb der Bohrung (13) mit einer Laschenplatte (16) fest verbunden, in die die Hülse (4) einmündet. Die Laschenplatte (16) ist über Knochenschrauben (17, 18) mit dem Oberschenkelknochen (10) fest verbunden. Die Knochenschrauben (17, 18) drücken die Laschenplatte (16) gegen die Längsfläche (14). Um eine möglichst gute Anlage der Laschenplatte (16) auf der Längsfläche (14) zu erzielen, ist die Laschenplatte (16) in ihrer Formgebung der Längsfläche (14) weitgehend angepaßt. Sie weist einen gewölbten Querschnitt auf, dessen Wölbungsradius quer zu ihrer Längsrichtung verläuft und demjenigen der mit ihr verbundenen Längsfläche (14) angepaßt ist. Durch die Laschenplatte (16) erstrecken sich Bohrlöcher (19, 20), die zur Aufnahme der Knochenschrauben (17, 18) dienen. Die Bohrlöcher (19, 20) sind in Längsrichtung der Laschenplatte (16) nebeneinander und in einer Linie mit einer Bohrung (21) angeordnet, in der die Hülse (4) in der Laschenplatte (16) mündet. Dabei sind die Bohrlöcher (19, 20) in einer vom Hüftkopf (9) abgewandten Richtung untereinander unterhalb der Bohrung (21) vorgesehen. Es ist jedoch auch möglich, weitere Bohrlöcher (19, 20) in der Laschenplatte (16) vorzusehen und diese beidseits der Bohrung (21) anzubringen.

Die Hülse (4) verläuft zur Längsrichtung der Laschenplatte (16) unter einem Winkel (22), der sich den anatomischen Verhältnissen des Oberschenkelknochens (10) anpaßt. Entscheidend ist, daß die Bohrung (13) sich durch den Hüftkopf (9) und den Schenkelhals (12) bis zur Längsfläche (14) erstreckt und die Laschenplatte auf der Längsfläche (14) anliegt. Auf diese Weise liegen die Richtungen sowohl der Laschenplatte (16) als auch der Hülse (4) fest, die sich durch die Bohrung (13) erstrecken muß. Entsprechend diesen Richtungen ist der Winkel (22) festzulegen. Die Auflagefläche (11) spannt eine Ebene auf, auf der sich die Bohrung (13) senkrecht erhebt.

Zunächst wird die Bohrung (13) in den Oberschenkelknochen (10) eingebracht. Sodann wird die Hülse (4) in die Bohrung (13) eingeschoben, bis die mit der Hülse (4) fest verbundene Laschenplatte (16) fest auf dem Oberschenkelschaft (15) aufliegt. Sodann wird die Laschenplatte (16) mit Hilfe der Knochenschrauben (17, 18) mit em Oberschenkelschaft (15) verschraubt, so daß die Laschenplatte (16) fest auf der Längsfläche (14) aufliegt. Nunmehr wird der Bolzen (3) in die Bohrung (13) und die Hülse (4) eingeführt, so daß der Bolzen (3) auf der Innenauskleidung (5) geführt wird. Diese besteht zweckmäßigerweise aus Polyäthylen, auf dem der aus Edelstahl gefertigte Bolzen (3) gut gleitet. Sowohl der Kopfteil (1) als auch der Führungsteil (2) sind aus Edelstahl gefertigt.

Nunmehr wird Knochenzement auf die zuvor in den Hüftkopf (9) eingefräste Auflagefläche (11) aufgetragen und die Kappe (6) mit der Basis (8) fest auf die Auflagefläche (11) aufgedrückt, so daß die Kappe (6) auf den Hüftkopf (9) aufzementiert wird. Nunmehr kann bei einer auf die Gleitfläche (7) auftreffenden Wechselbelastung sich der Bolzen (3) innerhalb der Hülse (4) je nach der Stärke der auftreffenden Belastung verschieben. Die dabei auftretenden Kräfte werden über den Hüftkopf in den Schenkelhals (12) und von diesem in den Oberschenkelschaft (15) eingeleitet.

## Patentansprüche

1. Hüftgelenkprothese mit einer Kappe (6), die eine in einer Hüftpfanne verschwenkbar lagerbare Gleitfläche (7) aufweist und mit der Kräfte auf einen der Hüftpfanne zugewandten Hüftkopf (9) eines Oberschenkelknochens (10) übertragbar sind, dadurch gekennzeichnet, daß die Kappe (6) eine der Gleitfläche (7) abgewandt angeordnete und auf einem Hüftkopf (9) eines Oberschenkelknochens (10) abstützbare Basis (8) aufweist und eine in Richtung der von der Kappe (6) zu übertragenden Kräfte nachgiebige Verankerung aufweist, die als ein Bolzen (3) ausgebildet ist, der in einer sich in Richtung der zu übertragenden Kräfte verlaufenden Führung verschieblich gelagert ist.

2. Hüftgelenkprothese nach Anspruch 1, dadurch gekennzeichnet, daß die Führung als eine im Hüftkopf (9) anordenbare und sich in Richtung der zu übertragenden Kräfte erstrekkende Hülse (4) ausgebildet ist.

3. Hüftgelenkprothese nach Anspruch 2, dadurch gekennzeichnet, daß die Hülse (4) von einer sich durch den Hüftkopf (9) erstreckenden Bohrung (13) aufnehmbar ausgebildet ist.

4. Hüftgelenkprothese nach Anspruch 3, dadurch gekennzeichnet, daß die Hülse (4) in der Bohrung (13) befestigbar ausgebildet ist.

5. Hüftgelenkprothese nach Anspruch 2 bis 4, dadurch gekennzeichnet, daß die Hülse (4) mit ihrem dem Bolzen (3) abgewandten Ende in einer Halterung befestigt ist, die mit dem Oberschenkelknochen (10) verbindbar ausgebildet ist.

6. Hüftgelenkprothese nach Anspruch 5, dadurch gekennzeichnet, daß die Halterung als eine mit dem Oberschenkelknochen (10) verbindbare Laschenplatte (16) ausgebildet ist.

7. Hüftgelenkprothese nach Anspruch 6, dadurch gekennzeichnet, daß die Laschenplatte (16) in Längsrichtung des Oberschenkelknochens (10) anordbar ausgebildet ist und in ihrer Gestalt einer Oberfläche des Oberschenkelknochens (10) angepaßt ist, mit der sie verbindbar ist.

8. Hüftgelenkprothese nach Anspruch 6 und 7, dadurch gekennzeichnet, daß die Laschenplatte (16) im Bereich einer dem Hüftkopf (9) gegenüberliegenden Längsfläche (14) des Oberschenkelknochens (10) anordbar ist und mindestens ein Bohrloch (19, 20) für die Aufnahme einer Knochenschraube (17, 18) aufweist, die durch das Bohrloch (19, 20) in den Oberschenkelknochen (10) einführbar ist.

9. Hüftgelenkprothese nach Anspruch 8, dadurch gekennzeichnet, daß die Laschenplatte (16) einen gewölbten Querschnitt aufweist, dessen Wölbungsradius quer zu ihrer Längsrichtung verläuft und demjenigen der mit ihr verbindbaren Längsfläche (14) angepaßt ist.

10. Hüftgelenkprothese nach Anspruch 8 und 9, dadurch gekennzeichnet, daß die Hülse (4) und die Bohrlöcher (19, 20) in Längsrichtung der Laschenplatte (16) nebeneinander angeordnet sind.

11. Hüftgelenkprothese nach Anspruch 10, dadurch gekennzeichnet, daß die Bohrlöcher (19, 20) sich in Richtung auf eine dem Oberschenkelknochen (10) zugewandt angeordnete Anlagefläche der Laschenplatte (16) beidseits einer Einmündung der Hülse (4) in die Laschenplatte (16) erstrecken.

12. Hüftgelenkprothese nach Anspruch 11, dadurch gekennzeichnet, daß die Bohrlöcher (19, 20) im Bereich eines der Kappe (6) abgewandt angeordneten Endes der Laschenplatte (16) vorgesehen sind und die Hülse (4) unmittelbar an deren anderem Ende in die Laschenplatte (16) einmündet.

13. Hüftgelenkprothese nach Anspruch 2 bis 12, dadurch gekennzeichnet, daß die Hülse (4) mit einer Innenauskleidung (5) versehen ist, auf der der Bolzen (3) gleitet.

14. Hüftgelenkprothese nach Anspruch 13, dadurch gekennzeichnet, daß die Innenauskleidung (15) aus Polyäthylen besteht.

15. Hüftgelenkprothese nach Anspruch 2 bis 14, dadurch gekennzeichnet, daß die Hülse (4) unter einem Winkel in die Laschenplatte (16) einmündet, der einem üblichen Winkel entspricht, der zwischen der Längsachse der Bohrung (13) einerseits und der Längsfläche (14), auf der die Laschenplatte (16) befestigbar ist, andererseits aufgespannt ist.

16. Hüftgelenkprothese nach Anspruch 1 bis 15, dadurch gekennzeichnet, daß die Basis (8) auf einer an den Hüftkopf (9) angefrästen Auflagefläche (11) aufliegbar ausgebildet ist.

17. Hüftgelenkprothese nach Anspruch 1 bis 16, dadurch gekennzeichnet, daß die Basis (8) auf die Auflagefläche (11) aufzementierbar ausgebildet ist.

18. Hüftgelenkprothese nach Anspruch 1 bis 17, dadurch gekennzeichnet, daß die Kappe (6) als Ausschnitt einer Hohlkugel ausgebildet ist.

19. Hüftgelenkprothese nach Anspruch 18, dadurch gekennzeichnet, daß der Bolzen (3) innerhalb der Hohlkugel befestigt ist und aus der Basis (8) herausragt.

20. Hüftgelenkprothese nach Anspruch 1 bis 19, dadurch gekennzeichnet, daß der Bolzen (3) einen kreisrunden Querschnitt aufweist.

21. Hüftgelenkprothese nach Anspruch 1 bis 19, dadurch gekennzeichnet, daß der Bolzen (3) einen eckigen Querschnitt aufweist.

**Claims**

1. A hip joint prosthesis comprising a cap (6) which has a sliding surface (7) which can be mounted pivotably in a hip socket and with which forces can be transmitted to a hip head (9), which is towards the hip socket, of a thigh bone (10), characterised in that the cap (6) has a base (8) which is arranged facing away from the sliding surface (7) and which can be supported against a hip head (9) of a thigh bone (10), and the cap has an anchorage which is yielding in the direction of the forces to be transmitted by the cap (6) and which is in the form of a pin (3) which is mounted displaceably in a guide extending in the direction of the forces to be transmitted.

2. A hip joint prosthesis according to claim 1 characterised in that the guide is in the form of a sleeve (4) which can be arranged in the hip head (9) and which extends in the direction of the forces to be transmitted.

3. A hip joint prosthesis according to claim 2 characterised in that the sleeve (4) is adapted to be received by a bore (13) extending through the hip head (9).

4. A hip joint prosthesis according to claim 3 characterised in that the sleeve (4) is adapted to be fixed in the bore (13).

5. A hip joint prosthesis according to claims 2 to 4 characterised in that the sleeve (4) is fixed by its end remote from the pin (3) in a holding means which is adapted to be connected to the thigh bone (10).

6. A hip joint prosthesis according to claim 5 characterised in that the holding means is in the form of a fixing plate (16) which can be connected to the thigh bone (10).

7. A hip joint prosthesis according to claim 6 characterised in that the fixing plate (16) is adapted to be arranged in the longitudinal direction of the thigh bone (10) and is matched in regard to its configuration to a surface of the thigh bone (10) to which it can be connected.

8. A hip joint prosthesis according to claim 6 and claim 7 characterised in that the fixing plate (16) can be arranged in the region of a longitudinal surface (14) of the thigh bone (10), which is in opposite relationship to the hip head (9), and has at least one bore hole (19, 20) for accommodating a bone screw (17, 18) which can be introduced through the bore hole (19, 20) into the thigh bone (10).

9. A hip joint prosthesis according to claim 8 characterised in that the fixing plate (16) is of a curved cross-section, the radius of curvature of which extends transversely with respect to its longitudinal direction and is adapted to that of the longitudinal surface (14) which can be connected thereto.

10. A hip joint prosthesis according to claim 8 and claim 9 characterised in that the sleeve (4) and the bore holes (19, 20) are arranged in juxtaposed relationship in the longitudinal direction of the fixing plate (16).

11. A hip joint prosthesis according to claim 10 characterised in that the bore holes (19, 20) extend towards a contact surface of the fixing plate (16) which is arranged towards the thigh bone (10), on both sides of a mouth opening of the sleeve (4) into the fixing plate (16).

12. A hip joint prosthesis according to claim 11 characterised in that the bore holes (19, 20) are provided in the region of an end of the fixing plate (16), which is arranged remote from the cap (6), and the sleeve (4) opens into the fixing plate (16) directly at the other end thereof.

13. A hip joint prosthesis according to claims 2 to 12 characterised in that the sleeve (4) is provided with an inner lining (5) on which the pin (3) slides.

14. A hip joint prosthesis according to claim 13 characterised in that the inner lining (15) comprises polyethylene.

15. A hip joint prosthesis according to claims 2 to 14 characterised in that the sleeve (4) opens into the fixing plate (16) at an angle which corresponds to a normal angle which is defined between the longitudinal axis of the bore (13) on the one hand and the longitudinal surface (14) to which the fixing plate (16) can be fixed, on the other hand.

16. A hip joint prosthesis according to claims 1 to 15 characterised in that the base (8) is adapted to bear against a support surface (11) which is milled on the hip head (9).

17. A hip joint prosthesis according to claims 1 to 16 characterised in that the base (8) is adapted to be cemented on to the support surface (11).

18. A hip joint prosthesis according to claims 1 to 17 characterised in that the cap (6) is in the form of a portion of a hollow sphere.

19. A hip joint prosthesis according to claim 18 characterised in that the pin (3) is fixed within the hollow sphere and projects out of the base (8).

**20.** A hip joint prosthesis according to claims 1 to 19 characterised in that the pin (3) is of round cross-section.

**21.** A hip joint prosthesis according to claims 1 to 19 characterised in that the pin (3) is of angular cross-section.

## Revendications

**1.** Prothèse de hanche comprenant une tête (6) qui présente une surface de glissement (7) pouvant être positionnée de manière basculante dans un cotyle et grâce à laquelle des forces peuvent être transmises à une tête fémorale (9) d'un fémur (10) tournée vers le cotyle, caractérisée en ce que la tête (6) présente une base (8) tournée à l'opposé de la surface de glissement (7) et pouvant être appuyée sur la tête fémorale (9), et un ancrage flexible dans le sens des forces à transmettre par la tête (6), ledit ancrage étant constitué par un boulon (3) qui est reçu, avec possibilité de déplacement, dans un guide (4) s'étendant dans le sens des forces à transmettre.

**2.** Prothèse de hanche selon la revendication 1, caractérisée en ce que le guide est constitué par une gaine (4) pouvant être disposée dans le tête fémorale (9) et s'étendant dans le sens des forces à transmettre.

**3.** Prothèse de hanche selon la revendication 2, caractérisée en ce que la gaine (4) est reçue par un canal (13) s'étendant à travers la tête fémorale (9).

**4.** Prothèse de hanche selon la revendication 3, caractérisée en ce que la gaine (4) est fixée dans le canal (13).

**5.** Prothèse de hanche selon l'une des revendication 2 à 4, caractérisée en ce que la gaine (4) est fixée par son extrémité opposée au boulon (3) à un dispositif de maintien relié au fémur (10).

**6.** Prothèse de hanche selon la revendication 5, caractérisée en ce que le dispositif de maintien est constitué par une plaque formant éclisse (16) pouvant être reliée au fémur (10).

**7.** Prothèse de hanche selon la revendication 6, caractérisée en ce que la plaque formant éclisse (16) est conformée de manière à être disposée dans le sens longitudinal du fémur (10) et à s'adapter à une surface du fémur (10) avec laquelle elle peut être reliée.

**8.** Prothèse de hanche selon l'une des revendications 6 et 7, caractérisée en ce que la plaque formant éclisse (16) peut être disposée contre une surface longitudinale (14) du fémur (10) faisant face à la tête fémorale (9) et présente au moins un taraudage (19,20) pour recevoir une vis à os (17,18) vissée dans le fémur (10).

**9.** Prothèse de hanche selon la revendication 8, caractérisée en ce que la plaque formant éclisse (16) présente une section transversale cintrée, dont le rayon de cintrage s'étend perpendiculairement à son sens longitudinal et est adapté à celui de la surface longitudinale (14) du fémur.

**10.** Prothèse de hanche selon l'une des revendications 8 et 9, caractérisée en ce que la gaine (4) et les alésages (19, 20) sont disposés les uns à côté des autres dans le sens longitudinal de la plaque formant éclisse (16).

**11.** Prothèse de hanche selon la revendication 10, caractérisée en ce que les alésage (19, 20) s'étendent en direction d'une surface de pose de la plaque formant éclisse (16) tournée vers le fémur (10), des deux côtés d'une embouchure de la gaine (4) dans la plaque formant éclisse (16).

**12.** Prothèse de hanche selon la revendication 11, caractérisée en ce que les alésages (19, 20) sont prévus dans la zone d'une extrémité de la plaque formant éclisse (16) disposée à l'opposé de la tête (6) et en ce que la gaine (4) débouche immédiatement à son autre extrémité dans la plaque formant éclisse.

**13.** Prothèse de hanche selon l'une des revendications 2 à 12, caractérisée en ce que la gaine (4) est munie d'un revêtement intérieur (5) sur lequel le boulon (3) glisse.

**14.** Prothèse de hanche selon la revendication 13, caractérisée en ce que le revêtement intérieur est en polyéthylène.

**15.** Prothèse de hanche selon l'une des revendications 2 à 14, caractérisée en ce que la gaine (4) débouche dans la plaque formant éclisse (16) en faisant avec celle-ci un angle correspondant à l'angle habituel formé entre l'axe longitudinal du canal (13) et la surface longitudinale (14) sur laquelle la plaque formant éclisse (16) est fixée.

**16.** Prothèse de hanche selon l'une des revendications précédentes, caractérisée en ce que la base (8) est en appui contre une surface de pose (11) fraisée sur la tête fémorale (9).

17. Prothèse de hanche selon l'une des revendications précédentes, caractérisée en ce que la base (8) est cimentée sur la surface de pose (11).

18. Prothèse de hanche selon l'une des revendications précédentes, caractérisée en ce que la tête (6) est en forme de portion de sphère creuse.

19. Prothèse de hanche selon la revendication 18, caractérisée en ce que le boulon (3) est fixé à l'intérieur de la tête et dépasse de la base (8).

20. Prothèse de hanche selon l'une des revendications précédentes, caractérisée en ce que le boulon (3) présente une section transversale circulaire.

21. Prothèse de hanche selon l'une des revendications 1 à 19, caractérisée en ce que le boulon (3) présente une section transversale polygonale.

Fig. 1

Fig.2